# EUROPEAN PATENT APPLICATION

(11) **EP 3 553 875 A1**
(43) Date of publication of application: **16.10.2019**
(21) Application number: 19167549.5
(22) Date of filing: 05.04.2019
(51) Int. Cl.: H01M 10/48, H01M 10/42

(54) **METHOD AND SYSTEM FOR DETECTING VOLATILE ORGANIC COMPOUNDS WITHIN A BATTERY ASSEMBLY**

(30) Priority: 11.04.2018 US 201862655946 P; 12.03.2019 US 201916351509
(71) Applicant: Astronics Advanced Electronic Systems Corp., Kirkland, WA 98034 (US)
(72) Inventor: Fifield, Jon, Kent, Washington 98031 (US)
(74) Representative: Loo, Chi Ching

(57) **Abstract**

Disclosed is a battery system (100) having a battery package assembly (101) including a battery cell (300) and a battery control circuit (230), wherein the battery control circuit (230) includes an internal volatile organic compound (VOC) sensor (210) and a control circuit (230). The battery cell (300) is connected to the battery control circuit (230), which is in turn connected to an external interface connection (102). The VOC sensor (210) is configured to detect the presence of a VOC within the battery package assembly (101) indicating a failure or impending failure the battery cell (300).

## Description

### FIELD OF THE DISCLOSURE

The subject matter of the present disclosure generally relates to monitoring battery assemblies' gaseous discharges, and more particularly relates to detecting volatile organic compounds (VOCs) within battery assemblies.

### BACKGROUND OF THE DISCLOSURE

Unexpected battery failures may lead to inconvenient and even dangerous circumstances, particularly when employed in enclosed environments or where flammable materials are in proximity.

Particularly, batteries employing lithium-ion (Li-ion) chemistry have had notable failures causing damage and fatalities. These can be particularly dangerous in aircraft when a failure occurs in overhead stowage bins or luggage storage areas, which has led to a ban on Li-ion batteries in checked baggage. These batteries are also however the most widely utilized battery type in portable electronic devices around the world. Li-ion battery failures may occur regardless of power density capacity, physical size or the types of personal, commercial or industrial applications in which they are employed. Even with existing industrial design standards and various regimes of government regulations concerning appropriate construction, manufacturing and safe operation of various battery chemistries, such as Li-ion battery technologies, failures of such batteries continue to occur. Li-ion is a generic term, covering several types of lithium battery chemistries and several battery assemble formats for various applications.

A battery includes three parts: an anode (-), a cathode (+), and the electrolyte - which can be made in a solid or liquid forms. Liquid organic electrolytes have high volatility and flammability, and elevated voltages and/or temperatures result with the electrolytes reacting with the active battery materials to release heat and gaseous by products. For example, the byproducts of a Li-ion battery during an out gassing are: methane, carbon monoxide, carbon dioxide, ethylene, and fluoroethane, as well as some other gasses.

The failure cycle of a battery involves three phases. The first phase includes an increase in battery temperature, and VOC gaseous discharge begins to occur. The second phase is labeled as "thermal runaway;" an event where the battery contains increasing amounts of heat to the point where it no longer requires any input power to continue to increase in temperature (self-heating) alone, which also increases the pressure within the cell. The third phase is where chemicals in the battery ignite, causing a catastrophic failure.

Most rechargeable battery systems consist of a battery cell(s), control and protection circuity, which includes firmware and software applications, and an interface connection. This system is usually packaged into one assembly with the interfacing connector located as part of the assembly body or a loose electrical connector. The battery cell is physically located as close as possible to the control and protection circuity board, to reduce line losses, and a membrane surrounding the system is included to protect the general public from direct contact of these parts.

There are certain existing protection methods that may be employed to reduce the hazard associated with failure mechanisms for various battery chemistries. For example, there are certain industry standards concerning potentially dangerous conditions in batteries such as overvoltage, overcurrent, over-charge, over-discharge and temperature.

The subject matter of the present disclosure is directed to overcoming, or at least reducing the effects of, one or more of the problems set forth above.

### BRIEF SUMMARY OF THE DISCLOSURE

Disclosed is a system and method for protecting a rechargeable battery system assembly from catastrophic failure due to the outgassing of a battery cell or package, by employing a volatile organic compound (VOC) senor configured to detect multi-gas or a singular carbon-based compound produced by a failing or failed battery cell. The VOCs are the result of a gaseous, odorous discharge, which, in some embodiments, have a non-polar molecular mass between 30 and 300 g/mole.

VOCs are carbon-based chemicals, and may be, for example, any organic compound with an initial boiling point less than or equal to 250 °C, as measured at standard pressure of 101.3 kPa. The VOCs may exist in a gaseous form at ambient temperature. In an embodiment, an included VOC sensor detects excess levels of carbon-based chemicals. The VOC sensor may be tailored to specifically measure or monitor certain chemicals, based on the battery's environmental applications. The VOC sensor produces an analog or digital signal to the accompanied control and protection circuity for further processing. The sensor may optionally provide a function to physically disconnect the affected battery cell(s) from the system.

In certain embodiments, the VOC sensor may be integrated into an existing rechargeable battery system design, thereby providing an additional protection method to minimize the hazard associated with various battery failure mechanisms that result in the outgassing of harmful chemicals.

### BRIEF DESCRIPTION OF THE DRAWINGS

The foregoing summary, preferred embodiments, and other aspects of the present disclosure will be best understood with reference to a detailed description of specific embodiments, which follows, when read in conjunction with the accompanying drawings, in which:
Figure 1 depicts a functional block diagram view of a single VOC sensor in a battery system assembly.
Figure 2 depicts a sectional view of a physical layout of the embodiment of Figure 1.
Figure 3 depicts a block diagram of a multi-VOC sensor application.

Like reference numbers and designations in the various drawings indicate like elements.

### DETAILED DESCRIPTION

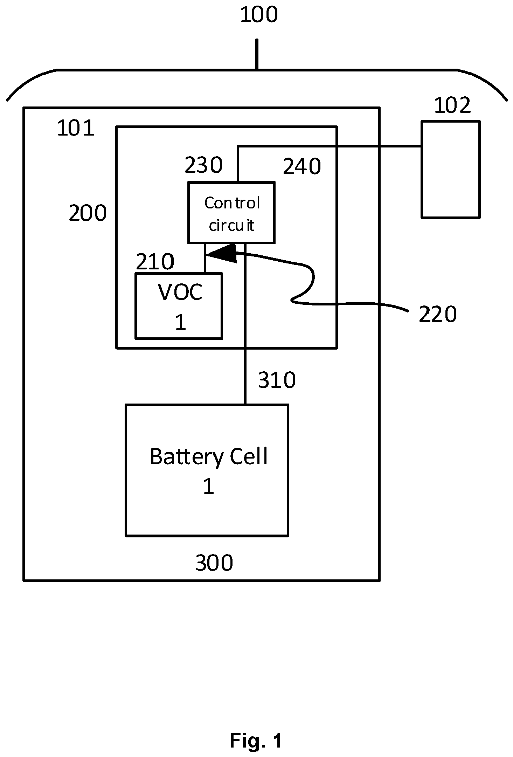

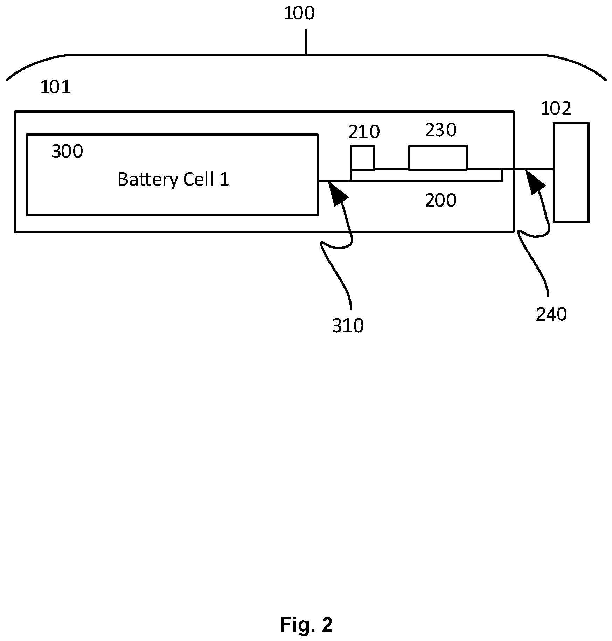

With reference to Figures 1-2, an embodiment incorporates a VOC sensor 210 into any existing rechargeable battery system design providing an additional protection method to minimize the hazard associated with various battery failure mechanisms, resulting in the outgassing of harmful chemicals.

VOC sensors 210 may be a Total VOC sensor (TVOC), which is a non-selective device that detects a wide variety of VOCs. In an embodiment, the TVOC may have a typical measurement level between 0 and 2000 ppm. The VOC sensor 210 may also be a targeted VOC sensor, which includes a specific correction factor used for sensor calibration. That is, a targeted VOC sensor may be used for a specific chemical, or for a subset of chemicals, and may be calibrated to detect for those particular VOC chemicals. A threshold value is configured for VOC concentration levels. For example, in a three-cell 60Wh battery assembly, a threshold level may be set to 90 or 100 ppb (parts per billion).

A battery system package layout 100 contains the major elements within a rechargeable battery system, excluding VOC sensor 210. Specifically, the battery package assembly 101 includes battery 300 and battery control circuitry 200. External interface connection 102 provides an interface to external circuitry systems for charging, discharging, monitoring, communicating and controlling a battery package 101. This is where the electrical energy is provided to charge the battery and also to discharge the battery.

The battery terminals 310 include positive and negative wired connections which are directly connected, typically by spot welding or a suitable mechanical attachment method, to the control and protection circuit board 230. In an embodiment, the connections between the battery 300 and control and protection circuit board 200 may include additional sensor connections. For example, a temperature sensor may be included to monitor inside the battery cell 300.

Control and protection block 230 includes a variety of functions, including rechargeable battery system applications. In some embodiments, control and protection block 230 may also function as a Microprocessor Control Unit (MCU)-type device. This circuit controls the energy flow to and from the battery and interface 102 via interface connection line 240. This circuit provides various protective functions for the battery system. Along with the power lines, contained in interface connection line 240, a status line can be provided to indicate a failure of the battery due to an output measurement value of VOC sensor 210, as an example.

VOC sensor 210 will measure elevated levels of VOCs in the environment within battery package 101. An elevated level may be a threshold limit value based on the existing inherited and predetermined VOC environmental condition for a battery system. An established threshold limit may utilize existing industrial standards, such as Occupational Exposures Limits (OELs) or Temporary Emergency Exposure Limits (TEELs) as a reference guide to set a threshold value. The measurement value from the VOC sensor 210 may produce a digital signal, and transmits it via wired system 220 to control circuit 230 for further processing. In another embodiment, VOC sensor 210 may produce an analog signal that is sent via wired system 220 to the control circuit 230 for further processing.

Within control circuit 230, the processing of the signal from VOC sensor 210 can be used in a variety of applications. For example, upon receiving an active signal from VOC sensor 210, due to a failure of battery cell 300, control circuit 230 could disconnect the positive and/or the negative leads of the battery 300 within battery control circuit 200. This then decouples battery 300 with interface connector 102, preventing any energy from being supplied to the failed battery cell, thereby reducing a thermal runaway event.

In another embodiment, VOC sensor 210 may be interfaced with an independent Microprocessor Unit (MPU) device. The sensor 210 may then be programmed for non-selective or targeted VOC measurement processing. The sensor 210 may then provide a digital or analog signal, and transmit it via wired system 220 to control circuit 230. It may then be connected to another microprocessor for further processing.

In another embodiment, within the control circuit 230, the processing of a signal from VOC sensor 210 may be sent as an external digital signal to outside the battery assembly 101, via interface connection line 240, through the external interface connection 102. The signal may indicate a hazard associated with a failure of battery 300. The external processing may be used, for example, to turn off the input power to the battery assembly 101.

The disclosed invention may be employed in detecting overheated electronic components inside other electronic devices, as certain electronic component failures may present themselves as an outgassing of a distressed part before complete failure and before other existing protection and monitoring systems could react to the stressed component.

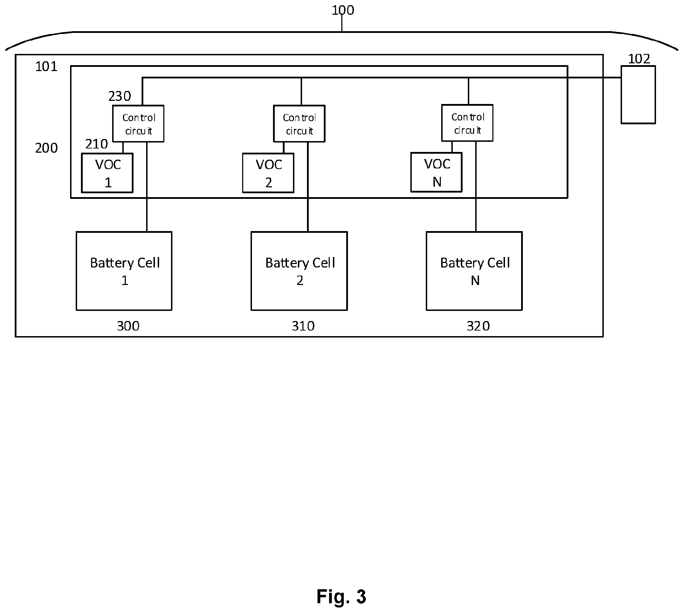

Figure 3 depicts a multi-cell embodiment, each battery cell having an associated control unit and VOC sensor.

In an embodiment, the VOC sensor 210 may be self-powered. For example, battery cell 300 may be used to power the VOC sensor itself, since any detection of excessive emission levels of VOCs from the battery 300 will occur before the battery cell 300 reaches dangerous levels, and will need to be shut down.

In an embodiment, physical disconnect of the battery cell 300 may be accomplished via an electrically controlled switch. The switch may be located between the input power supply and battery cell, which is controlled by the control circuit. The switch may also be located in another suitable location. The switch may then be toggled or switched to disconnect the battery cell and the input power supply, upon detection of an elevated or exceeded threshold level of VOCs.

In a further embodiment, battery cell 300 may be disconnected from the input power supply with a shut-off signal. The shut-off signal may be generated from the control circuit, upon detection of an elevated or exceeded threshold level of VOCs, which is then communicated from the battery system to an external control system. This results in an external disconnection of the input power.

Although the disclosed subject matter has been described and illustrated with respect to embodiments thereof, it should be understood by those skilled in the art that features of the disclosed embodiments can be combined, rearranged, etc., to produce additional embodiments within the scope of the invention, and that various other changes, omissions, and additions may be made therein and thereto, without parting from the spirit and scope of the present invention.

## Claims

1. A battery system (100), comprising:
a battery package assembly (101) including at least one battery cell (300) and a battery control circuit (230);
wherein the battery control circuit (200) further comprises an internal volatile organic compound (VOC) sensor (210) and a control circuit (230);
wherein the at least one battery cell (300) is connected to the battery control circuit (230); and
an external interface connection (102);
wherein the VOC sensor (210) is configured to detect the presence of a VOC within the battery package assembly (101) indicating a failure or impending failure of at least one of the at least one battery cell (300).

2. A system of claim 1 wherein the battery control circuit (200) is configured to receive from the VOC sensor (210) an indication that a predetermined threshold of VOC presence has been exceeded.

3. A system of claim 2 wherein the battery control circuit (200) is further configured to communicate to the external interface connection (102) an alert that the predetermined threshold of VOC presence has been exceeded.

4. A system of claim 2 wherein upon receipt by the battery control circuit (230) of the indication the battery control circuit (230) is configured to cause the at least one battery cell (300) to be disconnected.

5. A system of claim 1 wherein the internal VOC sensor (210) is in communication with the control circuit via a wired system (220).

6. A system of claim 1 wherein the external interface connection (102) is in communication with the control circuit (230) via an interface connection line (240).

7. A system of claim 1 wherein the at least one battery cell (300) is at least one lithium-ion battery cell.

8. A battery system (100), comprising:
a battery package assembly (101) including a plurality of battery cells (300) and battery control circuitry (200);
wherein the battery control circuitry (200) further comprises a plurality of internal volatile organic compound (VOC) sensors (210), each associated with one of the plurality of battery cells (300), and a plurality of control circuits (230), each associated with one of the plurality of battery cells (300);
wherein each of the control circuits (230) are each in communication with an external interface connection (102); and
wherein each VOC sensor (210) is configured to detect the presence of a VOC emanating from the battery cell (300) with which it is associated.

9. A system of claim 8 wherein the control circuit (230) is configured to receive from the VOC sensor (210) with which it is associated an indication that a predetermined threshold of VOC presence has been exceeded.

10. A system of claim 9 wherein the control circuit (230) receiving the indication that the predetermined threshold has been exceeded is further configured to communicate to the external interface connection (102) an alert regarding the battery cell (300) with which it is associated.

11. A system of claim 8 wherein the control circuit (230) receiving the indication that the predetermined threshold has been exceeded is further configured to cause the associated battery cell (300) to be disconnected.

12. A system of claim 8 wherein each VOC sensor (210) is in communication with its associated control circuit (230) via a wired system (220).

13. A system of claim 8 wherein each battery cell (300) is a lithium-ion battery cell.
